# EUROPÄISCHE PATENTSCHRIFT

(11) **EP 4 378 427 B1**
(45) Veröffentlichungstag und Bekanntmachung des Hinweises auf die Patenterteilung: **07.10.2026**
(21) Anmeldenummer: 23213627.5
(22) Anmeldetag: 01.12.2023
(51) Int. Cl.: A61F 2/32, A61F 2/38, A61F 2/40, A61F 2/42, A61F 2/44, A61F 2/36, A61F 2/30

(54) **MEDIZINISCHES GELENKABRIEBSAMMELVORRICHTUNGSIMPLANTAT, GELENKENDOPROTHESENKOMPONENTE UND GELENKENDOPROTHESE**
MEDICAL JOINT WEAR DEBRIS COLLECTION DEVICE IMPLANT, JOINT ENDOPROSTHESIS COMPONENT, AND JOINT ENDOPROSTHESIS
IMPLANT MÉDICALE DE COLLECTE DES DÉBRIS D'ABRASION D'ARTICULATION , COMPOSANT D'ENDOPROTHÈSE D'ARTICULATION ET ENDOPROTHÈSE D'ARTICULATION

(30) Priorität: 02.12.2022 DE 102022132066
(43) Veröffentlichungstag der Anmeldung: 05.06.2024
(73) Patentinhaber: Aesculap AG, 78532 Tuttlingen (DE)
(72) Erfinder: Jansen-Troy, Arne, 78333 Stockach (DE); Utz, Michael, 78532 Tuttlingen (DE); Maas, Allan, 78467 Konstanz (DE)
(74) Vertreter: Hoeger, Stellrecht & Partner Patentanwälte mbB

(56) Entgegenhaltungen:
- US-A- 5 378 228
- US-A- 5 879 406
- US-A1- 2005 055 101
- US-A1- 2011 276 146
- US-A1- 2021 338 440

## Beschreibung

Die vorliegende Erfindung ist in den Ansprüchen definiert und betrifft ein medizinisches Gelenkabriebsammelvorrichtungsimplantat, welches ausgebildet ist, um in eine Gelenkkapsel eines Bewegungsgelenks implantiert zu werden, welches Bewegungsgelenk mindestens eine erste künstliche Gelenkendoprothesenkomponente umfasst mit einer ersten Gelenkfläche zur Ausbildung des Bewegungsgelenks im Zusammenwirken mit einer zweiten Gelenkfläche einer zweiten künstlichen Gelenkendoprothesenkomponente oder einer natürlichen Gelenkfläche eines natürlichen Gelenkknochens eines Menschen oder eines Tieres, wobei das Gelenkabriebsammelvorrichtungsimplantat einen Implantatkörper umfasst, an welchem ein Sammelraum zum Aufnehmen von Partikeln, insbesondere von Abriebpartikeln, welche durch Abrieb an der mindestens einen ersten Gelenkendoprothesenkomponente in die in einer Gelenkkapsel des Bewegungsgelenks enthaltene Synovialflüssigkeit abgegeben werden, ausgebildet ist, wobei der Sammelraum einen Sammelraumeinlass und einen Sammelraumauslass umfasst und bis auf den Sammelraumeinlass und den Sammelraumauslass von einer fluiddichten, geschlossenen Sammelraumbegrenzungsfläche begrenzt ist, wobei der Sammelraumeinlass mit einem Sammelraumeinlassverschlusselement und/oder der Sammelraumauslass mit einem Sammelraumauslassverschlusselement verschlossen sind.

Ferner betrifft die vorliegende Erfindung eine Gelenkendoprothesenkomponente, welche ausgebildet ist in Form einer ersten Gelenkendoprothesenkomponente mit einer ersten Gelenkfläche zur Ausbildung eines Bewegungsgelenks im Zusammenwirken mit einer zweiten Gelenkfläche einer zweiten Gelenkendoprothesenkomponente oder einer natürlichen Gelenkfläche eines natürlichen Gelenkknochens eines Menschen oder eines Tieres.

Überdies betrifft die vorliegende Erfindung eine Gelenkendoprothese umfassend eine erste Gelenkendoprothesenkomponente und eine mit dieser zusammenwirkende zweite Gelenkendoprothesenkomponente zur Ausbildung eines Bewegungsgelenks.

Gelenkendoprothesen der eingangs beschriebenen Art kommen insbesondere bei Menschen und Tieren zum Einsatz, wenn bei einem natürlichen Gelenk eine oder beide zusammenwirkenden Gelenkflächen geschädigt sind. Die geschädigte natürliche Gelenkfläche wird zusammen mit einem Teil des Knochens, an dem sie ausgebildet ist, entfernt und durch eine entsprechend passende Gelenkendoprothesenkomponente ersetzt.

Derartige Gelenkendoprothesen können überall dort zum Einsatz kommen, wo natürliche Gelenke bewegungserhaltend durch künstliche Implantatteile ersetzt werden. Somit bilden die beschriebenen Gelenkendoprothesen bewegungserhaltende Endoprothesen.

Ein Problem bei allen Arten von Gelenkendoprothesen ist insbesondere, dass es zu Abrieb an den zusammenwirkenden Gelenkflächen kommt. Das Entstehen von Abrieb ist materialpaarungsunabhängig. Der Abrieb, bei dem es sich um feinste Partikel handeln kann, lagert sich insbesondere im Gewebe ab, welches das jeweils betroffene Gelenk umgibt. Eine Ablagerung kann sich auch noch weiter vom Gelenk weg erstrecken. Derartige Ablagerungen können zu weiterem Verschleiß der Gelenkflächen, mithin also der bewegungserhaltenden Implantatoberflächen, beitragen und zudem zu unerwünschten, pathologischen Körperreaktionen führen.

Ein weiteres Problem bei der Durchführung von chirurgischen Eingriffen zur Implantation von Gelenkendoprothesen ist insbesondere, dass dabei ebenfalls in unerwünschter Weise Partikel in das Gelenk eingebracht werden können, beispielsweise in Form von Abrieb von Sägeblättern. Überdies besteht auch die Gefahr, dass Knochenzementreste, welche insbesondere der Gelenkfläche einer Gelenkendoprothesenkomponente Schaden zufügen können, in das Gelenk eingebracht werden und dadurch ebenfalls einen Verschleiß der Gelenkendoprothese beschleunigen können.

In der US 5,378,228 sind Verfahren und Vorrichtungen zur Dekompression und Filtration von Gelenkflüssigkeit durch Sammeln von partikelförmigen Fremdkörpern beschrieben. Aus der US 2011/0276146 A1 ist ein Prothesenimplantat bekannt, welches eine Dichtungsanordnung und eine Kammer zum Aufnehmen von Fremdkörperteilchen umfasst. Eine künstliche Gelenkbioprothese zur Verringerung von Abnutzung ist in der US 5,879,406 beschrieben. Die US 2005/0055101 A1 offenbart Knieendoprothesen und Prothesen anderer Gelenke. Aus der US 2021/0338440 A1 ist ein orthopädisches Knieimplantatsystem mit kontrollierter Steifheit bekannt.

Es ist daher eine Aufgabe, ein medizinisches Gelenkabriebsammelvorrichtungsimplantat, eine Gelenkendoprothesenkomponente und eine Gelenkendoprothese bereitzustellen, welche eine verbesserte Körperverträglichkeit aufweisen.

Diese Aufgabe wird bei einem medizinischen Gelenkabriebsammelvorrichtungsimplantat der eingangs beschriebenen Art erfindungsgemäß dadurch gelöst, das Sammelraumeinlassverschlusselement und das Sammelraumauslassverschlusselement aus einem Verschlusselementmaterial ausgebildet sind, welches in der Synovialflüssigkeit löslich ist.

Das vorgeschlagene medizinische Gelenkabriebsammelvorrichtungsimplantat kann zwar nicht das Entstehen von Abrieb und damit von Partikeln verhindern, die in unerwünschter Weise im Gelenk eines Patienten freigesetzt werden. Es ist jedoch insbesondere geeignet, derartige Partikel aus der Synovialflüssigkeit des Gelenks aufzunehmen und zu sammeln, und zwar in definierter Weise. Hierfür dient der Sammelraum, welcher am Implantatkörper ausgebildet ist. In ihm können sich Partikel jedweder Art, insbesondere Abriebpartikel von Gelenkflächen, Knochenzementreste oder auch Abrieb von bei der Implantation von Gelenkendoprothesenkomponenten eingesetzten Werkzeugen und Instrumenten sammeln. Auf diese Weise lässt sich eine Gefahr vermindern, dass die in der Gelenkkapsel unerwünschten Partikel zu Entzündungsreaktionen im Körper des Patienten führen. Insbesondere kann es sich dabei um pathologische Erscheinungen in Form von unspezifischen Reaktionen des Immunsystems wie Granulomen, Wundheilungsstörungen und Ekzemen handeln. In schweren Fällen kann es sogar zu Schädigungen von Organen kommen. Bekannt ist auch, dass anhaltende Entzündungserscheinungen zum Abbau von Knochensubstanz führen können, was in unerwünschter Weise zu einer Lockerung der implantierten Gelenkendoprothesenkomponente führen kann. Diesen Gefahren wirkt das vorgeschlagene medizinische Gelenkabriebsammelvorrichtungsimplantat wie erläutert entgegen. Der Sammelraum umfasst einen Sammelraumeinlass und einen Sammelraumauslass und ist bis auf den Sammelraumeinlass und den Sammelraumauslass von einer fluiddichten, geschlossenen Sammelraumbegrenzungsfläche begrenzt. Eine solche Weiterbildung des Gelenkabriebsammelvorrichtungsimplantats ermöglicht insbesondere ein definiertes Aufnehmen und Sammeln von Partikeln aus der Synovialflüssigkeit des Gelenks im Sammelraum. Der Sammelraum ist also nicht von einer durchlässigen Begrenzung umgeben beziehungsweise definiert, sondern geschlossen, um das Austreten von gesammelten Partikeln aus dem Sammelraum in eine Umgebung des Gelenkabriebsammelvorrichtungsimplantats zu verhindern. Durch das Vorsehen eines Sammelraumeinlasses und eines Sammelraumauslasses kann insbesondere eine definierte Strömungsbewegung der Synovialflüssigkeit in der Gelenkkapsel vorgegeben werden. Partikel können so in definierter Weise aus der Synovialflüssigkeit entfernt und im Sammelraum gesammelt werden. Um postoperativ zu verhindern, dass der Sammelraum insbesondere mit Blut zugesetzt wird, ist es vorteilhaft, dass der Sammelraumeinlass mit einem Sammelraumeinlassverschlusselement und/oder der Sammelraumauslass mit einem Sammelraumauslassverschlusselement verschlossen sind. So ist nach einer Implantation des Gelenkabriebsammelvorrichtungsimplantats der Sammelraum zunächst davor geschützt, dass in unerwünschter Weise Blut oder Körpergewebeteile in den Sammelraum gelangen und diesen zusetzen oder verstopfen können. Vorteilhaft ist es, dass das Sammelraumeinlassverschlusselement und das Sammelraumauslassverschlusselement aus einem Verschlusselementmaterial ausgebildet sind, welches in der Synovialflüssigkeit löslich ist. Diese Ausgestaltung ermöglicht es insbesondere, den Sammelraum wie vorgeschlagen zunächst vollständig zu verschließen, um das Eindringen von Blut und größeren Gewebeteilen zu verhindern, wodurch der Sammelraum zugesetzt und unter Umständen dauerhaft verstopft werden könnte. Durch eine geeignete Wahl des Verschlusselementmaterials können sich die Sammelraumeinlassverschluss- und Sammelraumauslassverschlusselemente einige Zeit nach der Operation unter dem biochemischen Einfluss der Synovialflüssigkeit auflösen und dadurch den Sammelraumeinlass und den Sammelraumauslass freigeben, sodass Partikel aus der Synovialflüssigkeit in den Sammelraum gelangen und in diesem zurückgehalten werden können.

Vorzugsweise definiert der Sammelraumeinlass eine Sammelraumeinlassquerschnittsfläche, welche einen Wert in einem Bereich von etwa 1 mm² bis etwa 40 mm² aufweist. Insbesondere kann die Sammelraumeinlassquerschnittsfläche in einem Bereich von etwa 2 mm² bis etwa 10 mm² liegen. Den Sammelraumeinlass in einer solchen Größe auszubilden, kann insbesondere sicherstellen, dass Synovialflüssigkeit mit unerwünschten Partikeln zuverlässig in den Sammelraum gelangen kann, um in diesem die Partikel zurückzuhalten.

Günstigerweise definiert der Sammelraumauslass eine Sammelraumauslassquerschnittsfläche, welche einen Wert in einem Bereich von etwa 1 mm² bis etwa 40 mm² aufweist. Insbesondere kann die Sammelraumauslassquerschnittsfläche einen Wert in einem Bereich von etwa 2 mm² bis etwa 10 mm² aufweisen. Den Sammelraumauslass in einer Größe wie angegeben vorzusehen, ist insbesondere vorteilhaft, um ein undefiniertes Austragen von im Sammelraum aufgenommenen Partikeln aus dem Sammelraum heraus zu verhindern.

Vorzugsweise weist der Sammelraum einen Durchmesser in einem Bereich von etwa 2 mm bis etwa 7 mm auf. Insbesondere kann der Durchmesser in einem Bereich von etwa 3 mm bis etwa 5 mm liegen. So können insbesondere hinreichend kleine Gelenkabriebsammelvorrichtungsimplantate ausgebildet werden, die als eigenständige Implantate, also insbesondere separat von einer Gelenkendoprothese, in eine Gelenkkapsel implantiert werden können. Ferner lassen sich solche Gelenkabriebsammelvorrichtungsimplantate auch an Gelenkendoprothesen anordnen beziehungsweise ausbilden.

Auf einfache Weise lässt sich das Gelenkabriebsammelvorrichtungsimplantat ausbilden, wenn der Sammelraum hohlzylindrisch oder im Wesentlichen hohlzylindrisch ausgebildet ist. Insbesondere kann der Sammelraum in Form einer das Gelenkabriebsammelvorrichtungsimplantat durchsetzenden Bohrung ausgebildet sein. So kann beispielsweise an einer Gelenkendoprothesenkomponente eine Bohrung vorgesehen werden, die dann insbesondere einen Sammelraum definieren kann.

Gemäß einer weiteren bevorzugten Ausführungsform kann vorgesehen sein, dass der Sammelraum am Gelenkabriebsammelvorrichtungsimplantat derart angeordnet oder ausgebildet ist, um von der Synovialflüssigkeit infolge einer Bewegung des Bewegungsgelenks durchströmt zu werden. Insbesondere kann eine solche Durchströmung wahlweise unidirektional oder bidirektional ermöglicht werden. Die vorgeschlagene Weiterbildung ermöglicht es insbesondere, das Gelenk selbst zu nutzen, um in definierter Weise eine Durchströmung des Gelenkabriebsammelvorrichtungsimplantats zu bewirken. So können insbesondere Partikel zuverlässig in den Sammelraum gelangen und in diesem zurückgehalten werden. Insbesondere eine unidirektionale Durchströmung des Gelenkabriebsammelvorrichtungsimplantats ist vorteilhaft, da so ein gezieltes und gerichtetes Extrahieren von Partikeln im Sammelraum ermöglicht wird.

Günstig ist es, wenn das Gelenkabriebsammelvorrichtungsimplantat eine Partikelextraktionseinrichtung zum Extrahieren von Abriebpartikeln aus der Synovialflüssigkeit. Das Vorsehen einer solchen Partikelextraktionseinrichtung ermöglicht insbesondere das definierte Extrahieren, also das Entfernen, von unerwünschten Partikeln aus der Synovialflüssigkeit des Gelenks.

Eine besonders kompakte Ausbildung des Gelenkabriebsammelvorrichtungsimplantats kann insbesondere dadurch erreicht werden, dass die Partikelextraktionseinrichtung in einem Sammelraum angeordnet oder ausgebildet ist.

Partikel lassen sich aus der Synovialflüssigkeit einfach und sicher extrahieren, wenn die Partikelextraktionseinrichtung eine Filtereinrichtung und/oder eine strömungsmechanische Abscheideeinrichtung umfasst. Mit Filtereinrichtungen sowie strömungsmechanischen Abscheideeinrichtungen können in gezielter Weise Partikel aus der Synovialflüssigkeit extrahiert werden. Insbesondere können sie an unterschiedliche Größen und Arten von Partikeln angepasst werden, um einen möglichst hohen Grad der Extraktion von unerwünschten Partikeln aus der Synovialflüssigkeit eines Gelenks zu ermöglichen.

Einfach und zuverlässig lassen sich Partikel aus der Synovialflüssigkeit extrahieren, wenn die Abscheideeinrichtung in Form einer Trägheits- oder Umlenkabscheideeinrichtung ausgebildet ist. Eine solche Abscheideeinrichtung ist insbesondere derart gestaltet, dass Totwasserbereiche existieren, in denen sich die Synovialflüssigkeit nicht bewegt, sodass also eine Geschwindigkeit der in der Synovialflüssigkeit enthaltenen Partikel auf Null reduziert wird. Derart abgebremste Partikel verbleiben zwangsweise im Sammelraum.

Auf einfache Weise lässt sich eine strömungsmechanische Abscheideeinrichtung ausbilden, wenn sie eine Mehrzahl, insbesondere im Sammelraum angeordneten oder ausgebildeten, Umlenkelementen umfasst zum Ausbilden von Abscheidebereichen, in welchen eine Strömungsgeschwindigkeit der Synovialflüssigkeit durch den Sammelraum hindurch Null oder im Wesentlichen Null ist. Die beschriebenen Abscheidebereiche, auch als sogenannte Totwasserzonen bezeichnet, ermöglichen das Sammeln und Ablagern von Partikeln im Sammelraum, ohne dass eine Gefahr besteht, dass diese Partikel den Sammelraum wieder in unerwünschter Weise verlassen können. So lassen sich Ablagerungsbereiche für Partikel im Sammelraum in einfacher und definierter Weise vorgeben.

Vorzugsweise ist mindestens ein Teil der Mehrzahl von Umlenkelementen in Form von Vorsprüngen ausgebildet. Die Vorsprünge können insbesondere von der Sammelraumbegrenzungsfläche vorstehend angeordnet oder ausgebildet sein. So lässt sich das Gelenkabriebsammelvorrichtungsimplantat auf einfache Weise ausbilden. Durch die Vorsprünge können insbesondere die erläuterten Totwasserzonen ausgebildet werden, um Partikel aus der Synovialflüssigkeit zu extrahieren.

Um das Entfernen von Partikeln aus der Synovialflüssigkeit weiter zu verbessern, ist es günstig, wenn mindestens ein Teil der Mehrzahl von Umlenkelementen eine Partikelrückhaltestruktur aufweist oder mindestens ein Partikelrückhalteelement trägt oder umfasst. Beispielsweise können die Umlenkelemente eine aufgeraute Oberflächenstruktur aufweisen, an der sich Partikel verhaken und auf diese Weise festgehalten werden können.

Vorzugweise umfasst die Filtereinrichtung mindestens ein Filterelement, welches ausgebildet ist, um Partikel, insbesondere Abriebpartikel, zurückzuhalten. Das mindestens eine Filterelement kann insbesondere derart ausgebildet sein, dass es Poren aufweist mit einer Größe, die kleiner ist als Partikel, die typischerweise beim Abrieb von Gelenkflächen entstehen. So können derartige Abriebpartikel an dem mindestens einen Filterelement zuverlässig zurückgehalten werden.

Vorteilhafterweise weist das mindestens eine Filterelement eine Porengröße in einem Bereich von etwa 5 µm ECD (Equivalent Circular Diameter) bis etwa 100 µm ECD auf. Mit einem solchen Filterelement können Abriebpartikel insbesondere zuverlässig aus der Synovialflüssigkeit extrahiert werden.

Vorteilhaft ist es, wenn zwei oder mehr Filterelemente vorgesehen sind, die sich in ihren Porengrößen unterscheiden. Eine solche Weiterbildung ermöglicht es insbesondere, die zwei oder mehr Filterelemente gezielt so anzuordnen, dass zunächst Abriebpartikel mit großem Durchmesser extrahiert werden und durch ein weiteres Filterelement dann Abriebpartikel mit kleinerem Durchmesser. So kann insbesondere verhindert werden, dass größere Partikel Filterelemente mit kleineren Porengrößen in unerwünschter Weise zusetzen können.

Vorteilhaft ist es, wenn zwischen dem Sammelraumeinlass und dem Sammelraumauslass mehrere Filterelemente nacheinander angeordnet oder ausgebildet sind und wenn Porengrößen der nacheinander angeordneten Filterelemente ausgehend vom Sammelraumeinlass abnehmen. Eine solche Anordnung hat insbesondere den Vorteil, dass zunächst mit dem ersten, dem Sammelraumeinlass nachgeschalteten Filterelement die größten Abriebpartikel herausgefiltert werden. Alle Partikel, die dieses Filterelement durchströmen können, können dann in nachgeschalteten Filterelementen mit kleineren Porengrößen extrahiert werden. So lässt sich ein unerwünschtes Zusetzen von Filterelementen mit kleinen Porengrößen durch Partikel mit großem Partikeldurchmesser verhindern.

Günstigerweise ist das mindestens eine Filterelement aus einem Filterelementmaterial ausgebildet. Es kann sich dabei um denselben Werkstoff handeln, aus dem das Gelenkabriebsammelvorrichtungsimplantat ausgebildet ist. Das Filterelementmaterial kann sich jedoch auch von einem Material, aus dem der Implantatkörper ausgebildet ist, unterscheiden. So können insbesondere Eigenschaften von Teilen beziehungsweise Komponenten des Gelenkabriebsammelvorrichtungsimplantats optimiert werden.

Vorzugsweise ist das Gelenkabriebsammelvorrichtungsimplantat aus einem Gelenkabriebsammelvorrichtungsimplantatmaterial ausgebildet. Diese Ausgestaltung ermöglicht es insbesondere, zur Ausbildung des Implantatkörpers einen dafür optimal geeigneten Werkstoff einzusetzen.

Um hinreichend stabile Gelenkabriebsammelvorrichtungsimplantate ausbilden zu können, ist es vorteilhaft, wenn das Gelenkabriebsammelvorrichtungsimplantatmaterial ein Kunststoff und/oder ein metallischer Werkstoff ist.

Um das Gelenkabriebsammelvorrichtungsimplantat optimal ausbilden zu können ist es vorteilhaft, wenn das Gelenkabriebsammelvorrichtungsimplantatmaterial und das Filterelementmaterial durch unterschiedliche Werkstoffe gebildet sind. So lassen sich unterschiedliche gewünschte Eigenschaften des Gelenkabriebsammelvorrichtungsimplantats durch entsprechende Wahl der Werkstoffe optimieren.

Ferner kann es vorteilhaft sein, wenn das Gelenkabriebsammelvorrichtungsimplantat einen Wirkstoffbehälter umfasst, welcher mit einem medizinischen oder pharmazeutischen Wirkstoff befüllt oder befüllbar ist. Ein solcher Wirkstoff kann beim Durchströmen des Gelenkabriebsammelvorrichtungsimplantat beispielsweise kontinuierlich abgegeben werden, um eine entsprechende Wirkung in der Gelenkkapsel über einen längeren Zeitraum hinweg zu entfalten.

Vorzugsweise ist der Wirkstoff entzündungshemmend. So kann eine Gefahr minimiert werden, dass Partikel in der Gelenkkapsel zu Entzündungsreaktionen führen.

Gemäß einer weiteren bevorzugten Ausführungsform kann vorgesehen sein, dass eine innere Oberfläche des Sammelraums, insbesondere die Sammelraumbegrenzungsfläche, mit einer Beschichtung versehen ist zum chemischen oder elektrostatischen Binden von Abriebpartikeln. Eine solche Beschichtung hat insbesondere den Vorteil, dass Partikel auch dann zurückgehalten werden können, wenn diese nicht in die oben beschriebenen Totwasserzonen gespült oder durch Filterelemente zurückgehalten werden. So kann insbesondere eine gesamte innere Oberfläche des Sammelraums zum Rückhalten von Partikeln genutzt werden.

Günstig ist es, wenn das Gelenkabriebsammelvorrichtungsimplantat durch ein generatives Herstellungsverfahren ausgebildet ist. Insbesondere kann es durch 3D-Druck oder selektives Lasersintern ausgebildet sein. Derartige Herstellungsverfahren ermöglichen es insbesondere, auch komplexe und kleinteilige Strukturen definiert und hochpräzise auszubilden. Insbesondere lassen sich so beispielsweise Umlenkelemente von Abscheideeinrichtungen im Inneren des Sammelraums auch mit komplizierten geometrischen Gestaltungen ausbilden.

Die eingangs gestellte Aufgabe wird ferner bei einer Gelenkendoprothesenkomponente der eingangs beschriebenen Art erfindungsgemäß dadurch gelöst, dass die erste Gelenkendoprothesenkomponente mindestens ein, insbesondere nur ein, Gelenkabriebsammelvorrichtungsimplantat umfasst oder bildet, wie es oben in Form bevorzugter Ausführungsformen beschrieben wurde.

Eine solche Gelenkendoprothesenkomponente weist dann auch die bereits oben im Zusammenhang mit bevorzugten Ausführungsformen von Gelenkabriebsammelvorrichtungsimplantaten beschriebenen Vorteile auf. Ferner ermöglicht sie es insbesondere, nicht nur die Funktion zur Ausbildung eines Teils des Gelenks zu übernehmen, sondern auch Partikel in der oben beschriebenen Weise, die unerwünscht in der Synovialflüssigkeit enthalten sind, aus dieser zu extrahieren. So muss kein Gelenkabriebsammelvorrichtungsimplantat zusätzlich, also separat, zur Gelenkendoprothesenkomponente in die Gelenkkapsel implantiert werden, sondern das Gelenkabriebsammelvorrichtungsimplantat kann insbesondere in die Gelenkendoprothese integriert sein.

Vorteilhaft ist es, wenn das Gelenkabriebsammelvorrichtungsimplantat an der ersten Gelenkendoprothese räumlich getrennt von der ersten Gelenkfläche angeordnet oder ausgebildet ist. So kann insbesondere zuverlässig sichergestellt werden, dass insbesondere ein Sammelraumeinlass und ein Sammelraumauslass des Gelenkabriebsammelvorrichtungsimplantats für ein Durchströmen mit Synovialflüssigkeit dauerhaft frei zugänglich sind und nicht durch eine mit der ersten Gelenkfläche zusammenwirkende weitere Gelenkfläche einer zweiten Gelenkendoprothesenkomponente oder der Gelenkfläche eines natürlichen Knochens eines Patienten dauerhaft oder zeitweise verschlossen werden können.

Vorteilhaft ist es, wenn an der ersten Gelenkfläche weder ein Sammelraumeinlass noch ein Sammelraumauslass des Sammelraums angeordnet oder ausgebildet sind. So kann insbesondere verhindert werden, dass der Sammelraumauslass beziehungsweise der Sammelraumauslass durch eine weitere Gelenkendoprothesenkomponente oder eine Gelenkfläche eines natürlichen Knochens eines Patienten dauerhaft oder temporär verschlossen werden können.

Vorzugsweise sind der Sammelraumeinlass und der Sammelraumauslass außerhalb der ersten Gelenkfläche oder getrennt von dieser angeordnet oder ausgebildet. So kann Synovialflüssigkeit insbesondere zuverlässig durch den Sammelraumeinlass in den Sammelraum strömen, sodass in der Synovialflüssigkeit enthaltene Partikel in definierter Weise extrahiert und im Sammelraum zurückgehalten werden können.

Günstig ist es, wenn das Gelenkabriebsammelvorrichtungsimplantat an der ersten Gelenkendoprothesenkomponente in einem Bereich geringer mechanischer Belastung angeordnet oder ausgebildet ist. Diese Ausgestaltung ermöglicht es insbesondere, sicherzustellen, dass es durch das Vorsehen des Gelenkabriebsammelvorrichtungsimplantats zu keiner Schwächung der Gelenkendoprothesenkomponente kommen kann. So kann insbesondere trotz vorgesehenem Gelenkabriebsammelvorrichtungsimplantat eine Dauerfestigkeit der Gelenkendoprothesenkomponente zuverlässig sichergestellt werden.

Vorteilhaft ist es, wenn die erste Gelenkendoprothesenkomponente aus einem Kunststoff und/oder aus einem Metall ausgebildet ist. So können insbesondere Gelenkendoprothesenkomponenten in gewünschter Weise ausgebildet werden, und zwar mit einer Stabilität, die für einen dauerhaften Einsatz der Gelenkendoprothesenkomponente geeignet ist.

Günstigerweise ist die erste Gelenkendoprothesenkomponente durch ein generatives Herstellungsverfahren ausgebildet. Insbesondere kann sie durch 3D-Druck oder selektives Lasersintern ausgebildet sein. Eine solche Herstellung ermöglicht insbesondere eine einfache Integration des Gelenkabriebsammelvorrichtungsimplantats in die Gelenkendoprothesenkomponente.

Die eingangs gestellte Aufgabe wird ferner bei einer Gelenkendoprothese der eingangs beschriebenen Art erfindungsgemäß dadurch gelöst, dass die erste und/oder die zweite Gelenkendoprothesenkomponente in Form einer der oben beschriebenen bevorzugten Ausführungsformen von Gelenkendoprothesenkomponenten ausgebildet sind.

Die Gelenkendoprothese weist dann auch die bereits oben im Zusammenhang mit bevorzugten Ausführungsformen von Gelenkendoprothesenkomponenten beschriebenen Vorteile auf.

Vorzugsweise ist die Gelenkendoprothese in Form eines Kniegelenks, eines Hüftgelenks, eines Schultergelenks, eines Sprunggelenks, eines Handgelenks, eines Bandscheibengelenks oder eines Ellenbogengelenks ausgebildet. Die Gelenkendoprothese kann grundsätzlich in Form einer beliebigen bewegungserhaltenden Gelenkendoprothese ausgebildet sein, bei welcher mindestens zwei zusammenwirkende Gelenkflächen eine Gleitpaarung bilden, bei der ein Abrieb beim dauerhaften Einsatz unvermeidlich ist.

Die nachfolgende Beschreibung bevorzugter Ausführungsformen der Erfindung dient im Zusammenhang mit den Zeichnungen der näheren Erläuterung. Es zeigen:
- Figur 1:: eine schematische perspektivische, teilweise durchbrochene Gesamtansicht eines ersten Ausführungsbeispiels einer Gelenkendoprothese;
- Figur 2:: eine Schnittansicht längs Linie 2-2 in Figur 1;
- Figur 3:: eine schematische Schnittansicht eines ersten Ausführungsbeispiels eines Gelenkabriebsammelvorrichtungsimplantats;
- Figur 4:: eine schematische Schnittansicht eines weiteren Ausführungsbeispiels eines Gelenkabriebsammelvorrichtungsimplantats;
- Figur 5:: eine schematische Schnittansicht eines weiteren Ausführungsbeispiels eines Gelenkabriebsammelvorrichtungsimplantats;
- Figur 6:: eine schematische perspektivische, teilweise durchbrochene Ansicht eines Ausführungsbeispiels einer Gelenkendoprothesenkomponente in Form eines Prothesenschafts;
- Figur 7:: eine schematische perspektivische Gesamtansicht eines Ausführungsbeispiels einer Gelenkendoprothese in Form eines künstlichen Bandscheibengelenks;
- Figur 8:: eine schematische Darstellung einer typischen Verschleißkurve einer Gleitpaarung; und
- Figur 9:: eine schematische Darstellung eines weiteren Ausführungsbeispiels eines in einer Gelenkkapsel implantiertes Gelenkabriebsammelvorrichtungsimplantats.

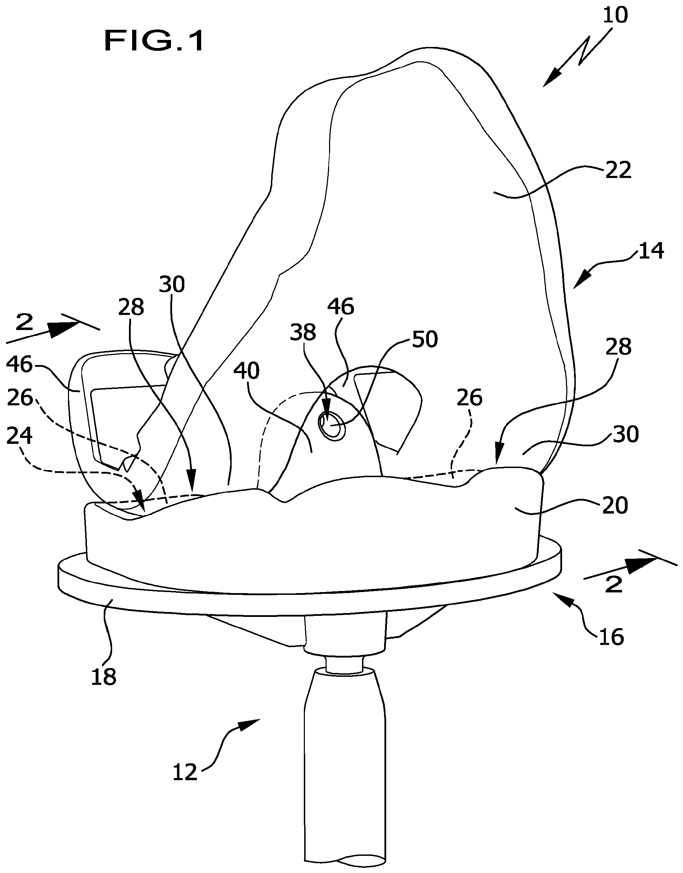

In Figur 1 ist beispielhaft ein erstes Ausführungsbeispiel einer Gelenkendoprothese 10 schematisch dargestellt.

Die Gelenkendoprothese 10 umfasst eine erste Gelenkendoprothesenkomponente 12 und eine mit dieser zusammenwirkende zweite Gelenkendoprothesenkomponente 14 zur Ausbildung eines Bewegungsgelenks. Das in Figur 1 dargestellte Ausführungsbeispiel der Gelenkendoprothese 10 ist in Form eines künstlichen Kniegelenks 16 ausgebildet.

Die erste Gelenkendoprothesenkomponente 12 umfasst eine an einer Tibia eines Patienten festlegbare Tibiakomponente 18 und eine auf dieser angeordnete Meniskuskomponente 20. Die zweite Gelenkendoprothesenkomponente 14 ist in Form einer an einem Femur festlegbaren Femurkomponente 22 ausgebildet.

Die erste Gelenkendoprothesenkomponente 12 umfasst eine erste Gelenkfläche 24 in Form einer an der Meniskuskomponente 20 ausgebildeten Gleitfläche 26, welche an einer korrespondierenden zweiten Gelenkfläche 28, welche durch zwei voneinander räumlich getrennte Kondylenflächen 30 der Femurkomponente 22 gebildet wird, anliegt. Die ersten und zweiten Gelenkflächen 24, 28 bilden eine zusammenwirkende Gleitpaarung. Die Gelenkflächen 24 und 28 sind ausgebildet, um aneinander abzugleiten und/oder aneinander abzurollen.

Die Gelenkendoprothese 10 ist nach der Implantation von einer Gelenkkapsel 66 umschlossen, die mit Synovialflüssigkeit 70 gefüllt ist.

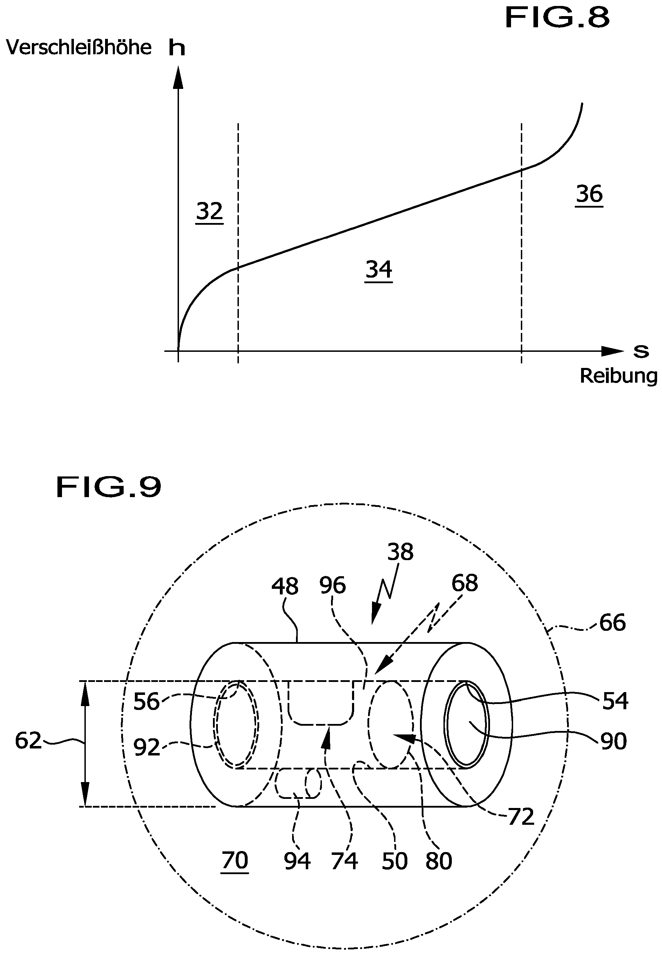

Bei einer Bewegung der Gelenkendoprothesenkomponenten 12 und 14 relativ zueinander kommt es zu einem praktisch unvermeidlichen Verschleiß. Figur 8 zeigt beispielhaft den Ablauf des auftretenden Verschleißes in den drei dargestellten Phasen.

Zunächst beginnt der Verschleiß mit einer Einlaufbereich 32, in welcher sich ein späteres Profil der zusammenwirkenden Gleitpartner herausbildet.

In einem sich daran anschließenden stationären Bereich herrscht ein über einen Reibweg s linearer Verschleiß vor. Der stationäre Bereich 34 ist auch der sogenannte Betriebsbereich einer Reibpaarung, da hier ein bekanntes Verhalten zugrunde liegt.

Ein Ausfallbereich 36 schließt sich an den stationären Bereich an. Hier tritt ein Ausfall der Reibpaarung auf. Der Ausfall kann beispielsweise durch Überschreiten einer zulässigen Verschleißhöhe h eintreten. Denkbar ist auch eine Zerstörung eines oder mehrerer der zusammenwirkenden Partner der Reibpaarung.

Insbesondere sowohl im Einlaufbereich 32 als auch im stationären Bereich 34 kommt es zu Abrieb an den zusammenwirkenden Gelenkflächen 24 und 28 und somit zum Entstehen von Partikeln unterschiedlicher Größe und unterschiedlicher Werkstoffe, die in die Synovialflüssigkeit 70 der Gelenkkapsel 66 abgegeben werden.

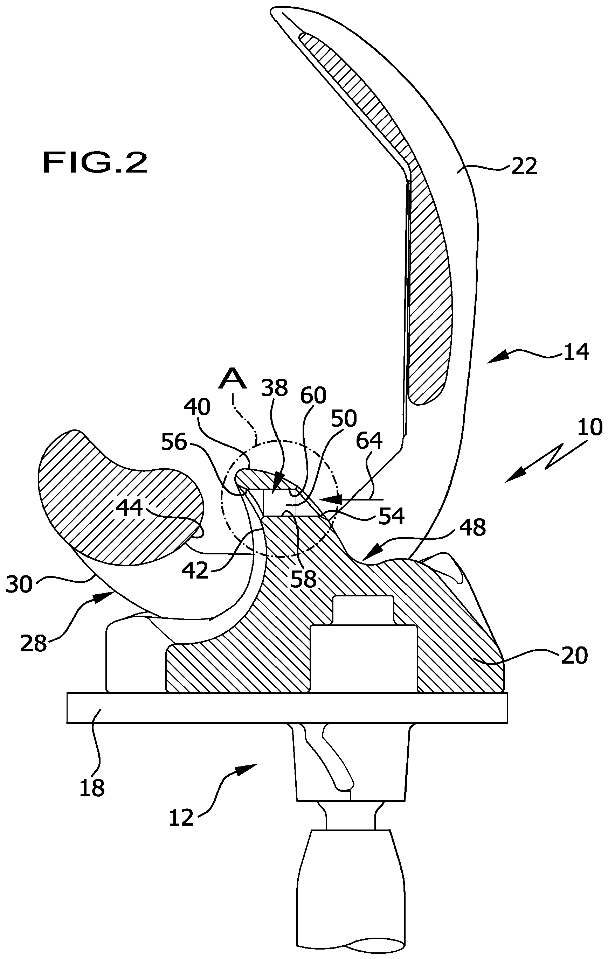

Um die beschriebenen Partikel aus der Synovialflüssigkeit 70 zu extrahieren, umfasst die Gelenkendoprothese 10, nämlich die erste Gelenkendoprothesenkomponente 12, ein medizinisches Gelenkabriebsammelvorrichtungsimplantat 38. Es ist bei dem Ausführungsbeispiel der Figuren 1 und 2 an der ersten Gelenkendoprothesenkomponente 12 in einem Bereich geringer mechanischer Belastung angeordnet beziehungsweise ausgebildet.

Bei der ersten Gelenkendoprothesenkomponente 12 des Ausführungsbeispiels der Figuren 1 und 2 ist das Gelenkabriebsammelvorrichtungsimplantat 38 an der von der ersten Gelenkendoprothesenkomponente 12 umfassten Meniskuskomponente 20 angeordnet beziehungsweise ausgebildet, und zwar an einem von der Meniskuskomponente 20 in Richtung auf die Femurkomponente 22 abstehenden Anschlag 40, welcher eine in posteriorer Richtung weisende Anschlagfläche 42 für einen Verbindungsabschnitt 44 an der Femurkomponente 22 aufweist, welcher Verbindungsabschnitt 44 zwei Kondylen 46, die die Kondylenflächen 30 umfassen, miteinander verbindet. Im Zusammenwirken verhindern der Verbindungsabschnitt 44 und der Anschlag 40 eine Bewegung der Femurkomponente 22 relativ zur Meniskuskomponente 20 in anteriorer Richtung, und zwar insbesondere bei einer Beugung des Kniegelenks 16.

Das Gelenkabriebsammelvorrichtungsimplantat 38 ist von der Meniskuskomponente 20 umfasst und umfasst somit einen Implantatkörper 48, welcher durch die Meniskuskomponente 20 selbst, mithin also durch die erste Gelenkendoprothesenkomponente 12, gebildet wird.

Am Implantatkörper 48 ist ein Sammelraum 50 ausgebildet zum Aufnehmen von Partikeln, insbesondere von beschriebenen Abriebpartikeln, welche durch Abrieb an der ersten Gelenkendoprothesenkomponente 12 in die in der Gelenkkapsel 66 des Bewegungsgelenks enthaltene Synovialflüssigkeit 70 abgegeben werden.

Der Sammelraum 50 umfasst einen Sammelraumeinlass 54 und einen Sammelraumauslass 56. Bis auf den Sammelraumeinlass 54 und den Sammelraumauslass 56 ist der Sammelraum 50 von einer fluiddichten, geschlossenen Sammelraumbegrenzungsfläche 58 begrenzt.

Der Sammelraumeinlass 54 definiert eine Sammelraumeinlassquerschnittsfläche. Diese weist bei dem in den Figuren 1 und 2 dargestellten Ausführungsbeispiel einen Wert in einem Bereich von etwa 1 mm² bis etwa 40 mm² auf. Insbesondere liegt der Wert der Sammelraumeinlassquerschnittsfläche in einem Bereich von etwa 2 mm² bis etwa 10 mm².

Der Sammelraumauslass 56 definiert in analoger Weise eine Sammelraumauslassquerschnittsfläche. Diese weist bei dem Ausführungsbeispiel der Figuren 1 und 2 einen Wert in einem Bereich von etwa 1 mm² bis etwa 40 mm² auf. Der Wert der Sammelraumauslassquerschnittsfläche liegt bei dem Ausführungsbeispiel insbesondere in einem Bereich von etwa 2 mm² bis etwa 10 mm².

In dem Ausführungsbeispiel der Figuren 1 und 2 ist der Sammelraum 50 hohlzylindrisch beziehungsweise im Wesentlichen hohlzylindrisch ausgebildet. Er ist in Form einer das Gelenkabriebsammelvorrichtungsimplantat 38 durchsetzenden Bohrung 60 ausgebildet.

Der Sammelraum 50 weist bei dem in Figuren 1 und 2 dargestellten Ausführungsbeispiel einen Durchmesser in einem Bereich von etwa 2 mm bis etwa 7 mm auf. Bei dem schematisch dargestellten Ausführungsbeispiel liegt der Durchmesser 62 in einem Bereich von etwa 3 mm bis etwa 5 mm.

Das Gelenkabriebsammelvorrichtungsimplantat 38, mithin also der Sammelraum 50, ist bei der Gelenkendoprothese 10 derart angeordnet beziehungsweise ausgebildet, dass er von der Synovialflüssigkeit 70 infolge einer Bewegung des Bewegungsgelenks durchströmt werden kann. Bei dem in den Figu-ren 1 und 2 schematisch dargestellten Ausführungsbeispiel ist eine Durchströmungsrichtung durch den Pfeil 64 symbolisiert. Infolge einer Bewegung der Gelenkendoprothesenkomponenten 12 und 14 relativ zueinander wird ein Syonvialflüssigkeitsstrom in Richtung des Pfeils 64 erzeugt, welcher durch den Sammelraumeinlass 54 in den Sammelraum 50 hinein- und durch den Sammelraumauslass 56 wieder aus diesem herausströmt.

In den Figuren 1 und 2 ist eine unidirektionale Durchströmung des Sammelraums 50 schematisch dargestellt. Bei in den Figuren nicht dargestellten Ausführungsbeispielen kann die Durchströmung des Sammelraums 50 mit Synovialflüssigkeit 70 auch bidirektional erfolgen.

Figur 9 zeigt ein weiteres Ausführungsbeispiel eines Gelenkabriebsammelvorrichtungsimplantats 38. Es ist als eigenständiges Implantat ausgebildet, um in eine Gelenkkapsel 66 eines in Figur 9 der Übersichtlichkeit wegen nicht dargestellten Bewegungsgelenks implantiert zu werden.

Das Gelenkabriebsammelvorrichtungsimplantat 38 umfasst einen Implantatkörper 48, an welchem ein Sammelraum 50 ausgebildet ist. Zur Bezeichnung des Gelenkabriebsammelvorrichtungsimplantats 38 sind dieselben Bezugszeichen für identische oder in ihrer Funktion ähnliche Teile beziehungsweise Komponenten des Gelenkabriebsammelvorrichtungsimplantats 38 wie beim Ausführungsbeispiel der Figuren 1 und 2 verwendet.

Beim Ausführungsbeispiel der Figur 9 ist der Implantatkörper 48 im Wesentlichen zylindrisch ausgebildet. Der Sammelraum 50 ist hohlzylindrisch ausgebildet mit einem Durchmesser 62.

Das Gelenkabriebsammelvorrichtungsimplantat 38 umfasst eine Partikelextraktionseinrichtung 68 zum Extrahieren von Partikeln 52 aus der Synovialflüssigkeit 70, die in der Gelenkkapsel 66 enthalten ist.

Die Partikelextraktionseinrichtung 68 ist im Sammelraum 50 angeordnet beziehungsweise ausgebildet.

Bei dem in Figur 9 beispielhaft dargestellten Ausführungsbeispiel umfasst die Partikelextraktionseinrichtung 68 eine Filtereinrichtung 72 sowie eine strömungsmechanische Abscheideeinrichtung 74.

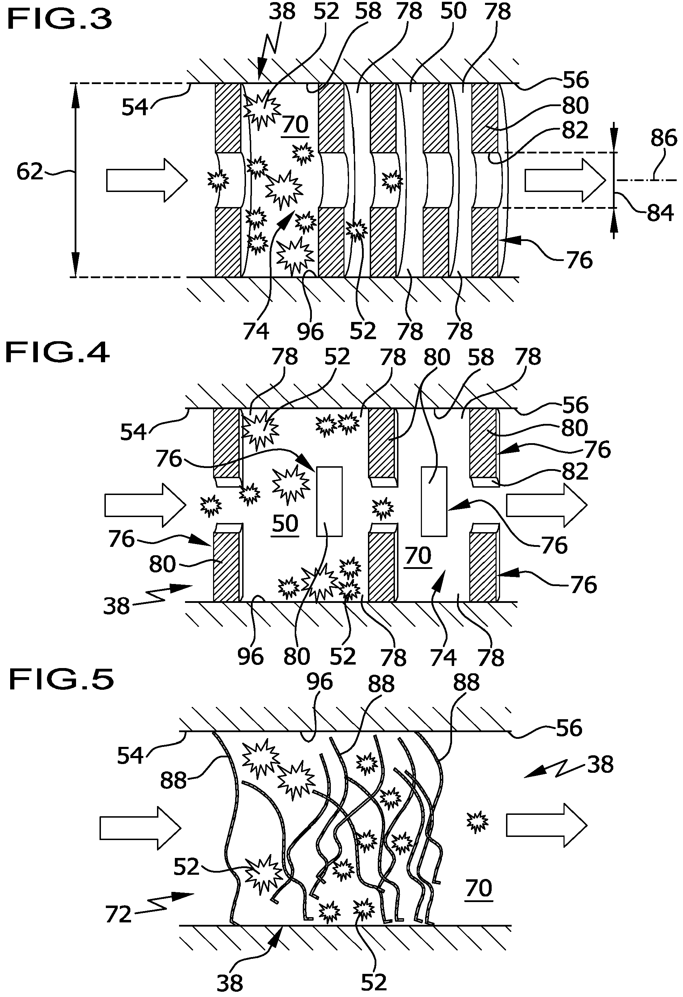

Der Aufbau der Abscheideeinrichtung 74 ist schematisch in Figur 3 in Form eines ersten Ausführungsbeispiels näher dargestellt.

Im Sammelraum 50 sind mehrere Umlenkelemente 76 der Abscheideeinrichtung 74 angeordnet. Sie dienen dem Zweck, Abscheidebereiche 78 auszubilden, in welchen eine Strömungsgeschwindigkeit der Synovialflüssigkeit 70 durch den Sammelraum 50 hindurch Null oder im Wesentlichen Null ist. Die Umlenkelemente 76 sind in Form von Vorsprüngen 80 ausgebildet. Die Vorsprünge 80 sind bei diesem Ausführungsbeispiel in Form von Ringvorsprüngen ausgebildet, die von der Sammelraumbegrenzungsfläche 58 vorstehen.

An jedem der Umlenkelemente 76 ist eine kreisförmige Durchbrechung 82 ausgebildet. Ein Durchmesser 84 der Durchbrechungen 82 weist einen Wert auf, der etwas geringer ist als ein Drittel des Durchmessers 62.

Beim Ausführungsbeispiel der Figur 3 sind die Durchbrechungen 82 koaxial zu einer Längsachse 86 des Sammelraums 50 ausgebildet.

Die Abscheidebereiche 78, auch als sogenannte Totwasserzonen bezeichnet, bilden sich zwischen benachbarten Umlenkelementen 76 nahe der Sammelraumbegrenzungsfläche 58 aus.

Figur 4 zeigt ein alternatives Ausführungsbeispiel einer Abscheideeinrichtung 74 im Sammelraum 50 eines Gelenkabriebsammelvorrichtungsimplantats 38. Auch hier sind mehrere Umlenkelemente 76 in Form von Vorsprüngen 80 im Sammelraum 50 angeordnet. Bei diesem Ausführungsbeispiel ergibt sich kein durchgehender geradliniger Strömungskanal wie beim Ausführungsbeispiel der Figur 3, der durch die koaxial zur Längsachse 86 ausgebildeten Durchbrechungen 82 in den Umlenkelementen 76 definiert wird.

Beim Ausführungsbeispiel der Figur 4 sind beispielhaft zwei Vorsprünge 80 derart angeordnet, dass eine geradlinige Durchströmung von Durchbrechungen 82 an Vorsprüngen 80, wie sie beim Ausführungsbeispiel bei Figur 3 vorgesehen sind, unmittelbar behindert wird. Synovialflüssigkeit 70 wird an den Vorsprüngen 80, die in einem Zwischenraum zwischen zwei Ringvorsprüngen angeordnet sind, um eine direkte Durchströmung der Durchbrechungen 82 zu verhindern, abgelenkt. Partikel 52 werden daher an den zentralen Vorsprüngen 80 umgelenkt und können so in Abscheidebereiche 78 nahe der Sammelraumbegrenzungsfläche 58 und der Ringvorsprünge gelangen. In diesen Abscheidebereichen 78 beträgt die Strömungsgeschwindigkeit der Synovialflüssigkeit 70 im Sammelraum 50 Null oder im Wesentlichen Null. Die Partikel 52 werden in den Abscheidebereichen 78 angereichert und können so nicht mehr aus dem Sammelraum 50 herausgelangen.

Die Filtereinrichtung 72 umfasst ein oder mehrere Filterelemente 88. Die Filterelemente 88 sind ausgebildet, um Partikel 52, insbesondere Abriebpartikel, zurückzuhalten.

Figur 5 zeigt schematisch ein Ausführungsbeispiel eines Gelenkabriebsammelvorrichtungsimplantats 38 mit einer Filtereinrichtung 72, welche mehrere Filterelemente 88 umfasst.

Die Filterelemente 88 weisen eine Porengröße in einem Bereich von etwa 5 µm ECD (Equivalent Circular Diameter) bis etwa 100 µm ECD auf.

Die mehreren Filterelemente 88 unterscheiden sich beim Ausführungsbeispiel der Figur 5 durch ihre jeweilige Porengröße. Beim Ausführungsbeispiel der Figur 5 sind zwischen dem Sammelraumeinlass 54 und dem Sammelraumauslass 56 mehrere Filterelemente 88 nacheinander angeordnet beziehungsweise ausgebildet, wobei Porengrößen der nacheinander angeordneten Filterelemente 88 ausgehend vom Sammelraumeinlass 54 abnehmen.

Optional können sowohl der Sammelraumeinlass 54 mit einem Sammelraumeinlassverschlusselement 90 als auch der Sammelraumauslass 56 mit einem Sammelraumauslassverschlusselement 92 verschlossen sein. Beim Ausführungsbeispiel der Figur 9 sind die beiden Verschlusselemente 90 und 92 jeweils gestrichelt eingezeichnet.

Das Sammelraumeinlassverschlusselement 90 und das Sammelraumauslassverschlusselement 92 sind aus einem Verschlusselementmaterial ausgebildet, welches in der Synovialflüssigkeit 70 löslich beziehungsweise lösbar ist. Dies ermöglicht es insbesondere, das Gelenkabriebsammelvorrichtungsimplantat 38 in die Gelenkkapsel 66 zu implantieren, wobei der Sammelraumeinlass 54 und der Sammelraumauslass 56 durch das Sammelraumeinlassverschlusselement 90 beziehungsweise das Sammelraumauslassverschlusselement 92 verschlossen sind. So wird verhindert, dass Blut oder größere Gewebeteile den Sammelraum 50 zusetzen und verstopfen können. Erst nach einer gewissen Zeit lösen sich die Verschlusselemente 90 beziehungsweise 92 vollständig auf und ermöglichen so ein Einströmen der Synovialflüssigkeit 70 in den Sammelraum 50.

Oberflächen der Umlenkelemente 76 können insbesondere eine in den Figuren nicht dargestellte Partikelrückhaltestruktur aufweisen beziehungsweise ein oder mehrere Partikelrückhalteelemente tragen oder umfassen. Auf diese Weise können Partikel 52 auch an einer Oberfläche der Umlenkelemente 76 anhaften und gegebenenfalls sogar chemische Bindungen eingehen, um ein Austreten der Partikel 52 aus dem Sammelraum 50 zu verhindern.

Die Abscheideeinrichtung 74 mit den Umlenkelementen 76 ist in Form einer Trägheits- oder Umlenkabscheideeinrichtung ausgebildet.

Die Umlenkelemente 76 sind einstückig, insbesondere monolithisch, mit dem Implantatkörper 48 ausgebildet. Sie können insbesondere aus einem Werkstoff ausgebildet sein, welcher mit dem den Implantatkörper 48 ausbildenden Werkstoff übereinstimmt.

Die Filterelemente 88 sind aus einem Filterelementmaterial ausgebildet. Der Implantatkörper 48 und damit ein wesentlicher Bestandteil des Gelenkabriebsammelvorrichtungsimplantats 38 ist aus einem Gelenkabriebsammelvorrichtungsimplantatmaterial ausgebildet. Bei dem in Figuren dargestellten Ausführungsbeispiel des Gelenkabriebsammelvorrichtungsimplantats 38, welches eine Filtereinrichtung 72 aufweist, sind das Gelenkabriebsammelvorrichtungsimplantatmaterial und das Filterelementmaterial durch unterschiedliche Werkstoffe gebildet. So kann der Implantatkörper 48 mit hinreichender Stabilität ausgebildet werden. Insbesondere kann er wie beschrieben durch die Gelenkendoprothesenkomponente 12 selbst gebildet sein. Die Filterelemente 88 der Filtereinrichtung 72 können auf diese Weise aus Werkstoffen ausgebildet werden, die eine optimierte Filtereigenschaft aufweisen. Insbesondere kann es sich beim Filterelementmaterial um ein Gewebe handeln. So lassen sich auch auf einfache Weise unterschiedliche Porengrößen bei Filterelementen realisieren.

Das Gelenkabriebsammelvorrichtungsimplantatmaterial kann insbesondere ein Kunststoff und/oder ein metallischer Werkstoff sein.

Das Gelenkabriebsammelvorrichtungsimplantat 38 kann optional einen Wirkstoffbehälter 94 umfassen. Dieser ist beispielhaft in Figur 9 punktiert eingezeichnet. Der Wirkstoffbehälter 94 ist optional mit einem medizinischen und/oder pharmazeutischen Wirkstoff befüllbar, beispielsweise einem entzündungshemmenden Wirkstoff. Der Wirkstoffbehälter 94 ist fluidwirksam mit dem Sammelraum 50 verbunden, sodass der im Wirkstoffbehälter 94 enthaltene Wirkstoff sukzessive in die den Sammelraum 50 durchströmende Synovialflüssigkeit 70 abgegeben werden kann.

Bei einem weiteren Ausführungsbeispiel ist optional eine innere Oberfläche 96 des Sammelraums 50, insbesondere also die Sammelraumbegrenzungsfläche 58, mit einer in den Figuren nicht dargestellten Beschichtung versehen zum chemischen und/oder elektrostatischen Binden von Partikeln 52. Durch eine solche Beschichtung kann eine Anhaftung und damit ein dauerhafter Verbleib von Partikeln 52 im Sammelraum 50 weiter verbessert werden.

Das in Figur 9 beispielhaft eigenständig dargestellte Gelenkabriebsammelvorrichtungsimplantat 38 kann wie bereits erläutert auch in die erste Gelenkendoprothesenkomponente 12 integriert sein, wie dies beim Ausführungsbeispiel 1 und 2 der Fall ist. Der Aufbau des Sammelraums 50 einschließlich einer Partikelextraktionseinrichtung 68 kann auch beim Ausführungsbeispiel der Figuren 1 und 2 wie oben in Verbindung mit den Figuren 3 bis 5 und 9 erläutert ausgebildet sein.

Wie beim Ausführungsbeispiel der Figuren 1 und 2 schematisch dargestellt ist das Gelenkabriebsammelvorrichtungsimplantat 38 an der ersten Gelenkendoprothesenkomponente 12 räumlich getrennt von der ersten Gelenkfläche 24 angeordnet beziehungsweise ausgebildet. Somit sind auch an der ersten Gelenkfläche 24 weder der Sammelraumeinlass 54 noch der Sammelraumauslass 56 eingeordnet beziehungsweise ausgebildet. Wie in den Figuren 1 und 2 schematisch dargestellt sind der Sammelraumeinlass 54 und der Sammelraumauslass 56 außerhalb der ersten Gelenkfläche 24 beziehungsweise getrennt von dieser angeordnet beziehungsweise ausgebildet.

Die Gelenkendoprothesenkomponente 12 ist bei den dargestellten Ausführungsbeispielen aus einem Kunststoff und/oder aus einem Metall ausgebildet.

Das Gelenkabriebsammelvorrichtungsimplantat 38 ist bei dem in Figur 9 dargestellten Ausführungsbeispiel durch ein generatives Herstellungsverfahren ausgebildet, nämlich entweder durch 3D-Druck oder durch selektives Lasersintern, je nachdem, ob der Implantatkörper 48 sowie die Abscheideeinrichtung 74 aus einem Kunststoff oder aus einem metallischen Werkstoff ausgebildet sind.

Die erste Gelenkendoprothesenkomponente 12 ist bei dem in Figur 1 und 2 beispielhaft dargestellten Ausführungsbeispiel aus einem Kunststoff sowie aus einem Metall ausgebildet. Die Meniskuskomponente 20 ist aus einem Kunststoff, die Tibiakomponente 18 aus einem Metall ausgebildet.

Auch die erste Gelenkendoprothesenkomponente 12 beziehungsweise deren Komponenten können durch ein generatives Herstellungsverfahren ausgebildet werden. Insbesondere ist dies mittels 3D-Druck oder selektivem Lasersintern möglich.

Die Ausgestaltung des medizinischen Gelenkabriebsammelvorrichtungsimplantats 38 ist nicht beschränkt auf konstruktive Ausgestaltungen wie sie insbesondere in Verbindung mit den Figuren 1 bis 5 und 9 erläutert wurden. Vielmehr können derartige Gelenkabriebsammelvorrichtungsimplantate 38 auch bei anderen Gelenkendoprothesen 10 vorgesehen sein, beispielsweise bei künstlichen Hüftgelenken, Schultergelenken, Sprunggelenken, Handgelenken, Ellenbogengelenken oder Bandscheibengelenken.

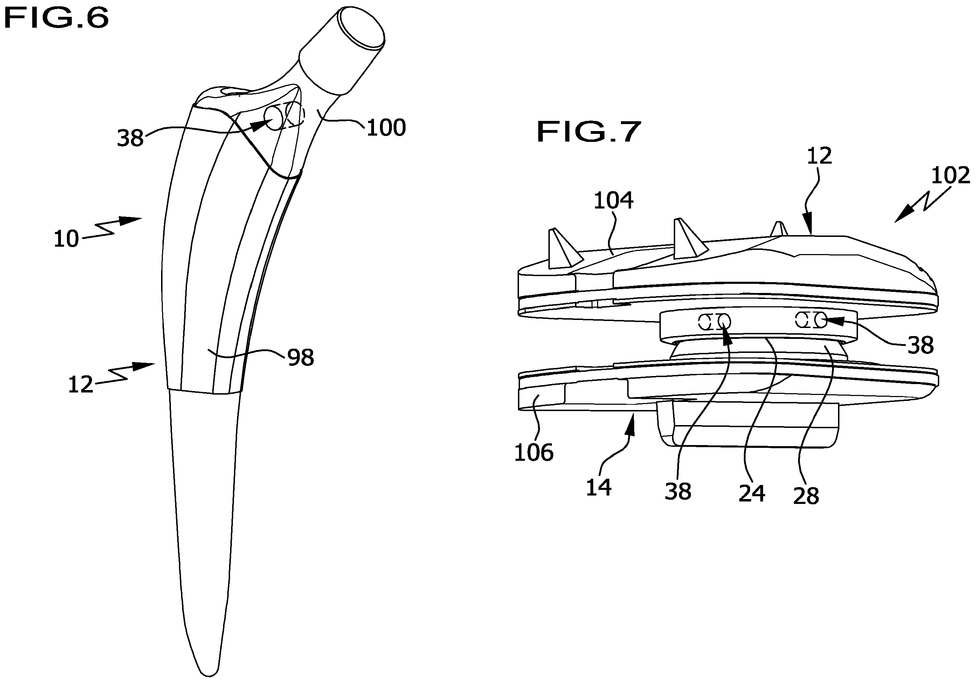

Figur 6 zeigt beispielhaft ein Ausführungsbeispiel eines Hüftschafts 98 eines künstlichen Hüftgelenks, an welchem im Bereich eines Halses 100 ein Gelenkabriebsammelvorrichtungsimplantat 38 angeordnet beziehungsweise ausgebildet ist. Es umfasst in diesem Ausführungsbeispiel eine Bohrung 60 zur Ausbildung des Sammelraums 50.

Ein Ausführungsbeispiel eines Bandscheibengelenks 102 ist schematisch in Figur 7 dargestellt. Es umfasst zwei Anlageelemente 104 und 106 zum Anlegen an benachbarte Wirbelkörper. Die Anlageelemente 104 und 106 bilden erste und zweite Gelenkendoprothesenkomponenten 12 und 14 mit ersten und zweiten zusammenwirkenden Gelenkflächen 24 und 28.

Beim Ausführungsbeispiel der Figur 7 sind zwei Gelenkabriebsammelvorrichtungsimplantate 38 vorgesehen, die an der ersten Gelenkendoprothesenkomponente 12, nämlich am Anlageelement 104, angeordnet beziehungsweise ausgebildet sind. Die konkrete Ausgestaltung der Gelenkabriebsammelvorrichtungsimplantate 38 bei den Ausführungsbeispielen der Figuren 6 und 7 ist entsprechend den bereits oben beschriebenen Ausführungsbeispielen von Gelenkabriebsammelvorrichtungsimplantaten 38 realisiert, sodass auf obige Beschreibung verwiesen werden kann.

Wie eingehend erläutert ist es mit den beschriebenen Ausführungsbeispielen von Gelenkabriebsammelvorrichtungsimplantaten 38 möglich, Partikel 52, insbesondere Abriebpartikel von ersten und/oder Gelenkflächen 24, 28 von ersten und zweiten Gelenkendoprothesenkomponenten 12, 14, aus der in der Gelenkkapsel 66 enthaltenen Synovialflüssigkeit 70 zu extrahieren und im Sammelraum 50 dauerhaft zurückzuhalten. So können etwaige Abstoßungsreaktionen im Körper eines Patienten zuverlässig und dauerhaft vermieden werden.

### Bezugszeichenliste

- 10: Gelenkendoprothese
- 12: erste Gelenkendoprothesenkomponente
- 14: zweite Gelenkendoprothesenkomponente
- 16: Kniegelenk
- 18: Tibiakomponente
- 20: Meniskuskomponente
- 22: Femurkomponente
- 24: erste Gelenkfläche
- 26: Gleitfläche
- 28: zweite Gelenkfläche
- 30: Kondylenfläche
- 32: Einlaufbereich
- 34: stationärer Bereich
- 36: Ausfallbereich
- 38: Gelenkabriebsammelvorrichtungsimplantat
- 40: Anschlag
- 42: Anschlagfläche
- 44: Verbindungsabschnitt
- 46: Kondyle
- 48: Implantatkörper
- 50: Sammelraum
- 52: Partikel
- 54: Sammelraumeinlass
- 56: Sammelraumauslass
- 58: Sammelraumbegrenzungsfläche
- 60: Bohrung
- 62: Durchmesser
- 64: Pfeil
- 66: Gelenkkapsel
- 68: Partikelextraktionseinrichtung
- 70: Synovialflüssigkeit
- 72: Filtereinrichtung
- 74: Abscheideeinrichtung
- 76: Umlenkelement
- 78: Abscheidebereich
- 80: Vorsprung
- 82: Durchbrechung
- 84: Durchmesser
- 86: Längsachse
- 88: Filterelement
- 90: Sammelraumeinlassverschlusselement
- 92: Sammelraumauslassverschlusselement
- 94: Wirkstoffbehälter
- 96: Oberfläche
- 98: Hüftschaft
- 100: Hals
- 102: Bandscheibengelenk
- 104: Anlageelement
- 106: Anlageelement

## Patentansprüche

1. Medizinisches Gelenkabriebsammelvorrichtungsimplantat (38), welches ausgebildet ist, um in eine Gelenkkapsel (66) eines Bewegungsgelenks implantiert zu werden, welches Bewegungsgelenk mindestens eine erste künstliche Gelenkendoprothesenkomponente (12) umfasst mit einer ersten Gelenkfläche (24) zur Ausbildung des Bewegungsgelenks im Zusammenwirken mit einer zweiten Gelenkfläche (28) einer zweiten künstlichen Gelenkendoprothesenkomponente (14) oder einer natürlichen Gelenkfläche eines natürlichen Gelenkknochens eines Menschen oder eines Tieres, wobei das Gelenkabriebsammelvorrichtungsimplantat (38) einen Implantatkörper (48) umfasst, an welchem ein Sammelraum (50) zum Aufnehmen von Partikeln (52), insbesondere von Abriebpartikeln, welche durch Abrieb an der mindestens einen ersten Gelenkendoprothesenkomponente (12) in die in einer Gelenkkapsel (66) des Bewegungsgelenks enthaltene Synovialflüssigkeit (70) abgegeben werden, ausgebildet ist, wobei der Sammelraum (50) einen Sammelraumeinlass (54) und einen Sammelraumauslass (56) umfasst und bis auf den Sammelraumeinlass (54) und den Sammelraumauslass (56) von einer fluiddichten, geschlossenen Sammelraumbegrenzungsfläche (58) begrenzt ist, wobei der Sammelraumeinlass (54) mit einem Sammelraumeinlassverschlusselement (90) und/oder der Sammelraumauslass (56) mit einem Sammelraumauslassverschlusselement (92) verschlossen sind, **dadurch gekennzeichnet, dass** das Sammelraumeinlassverschlusselement (90) und das Sammelraumauslassverschlusselement (92) aus einem Verschlusselementmaterial ausgebildet sind, welches in der Synovialflüssigkeit (70) löslich ist.

2. Medizinisches Gelenkabriebsammelvorrichtungsimplantat nach einem der voranstehenden Ansprüche, **dadurch gekennzeichnet, dass** der Sammelraum (50) hohlzylindrisch oder im Wesentlichen hohlzylindrisch ausgebildet ist, insbesondere in Form einer das Gelenkabriebsammelvorrichtungsimplantat (38) durchsetzenden Bohrung (60).

3. Medizinisches Gelenkabriebsammelvorrichtungsimplantat nach einem der voranstehenden Ansprüche, **dadurch gekennzeichnet, dass** der Sammelraum (50) am Gelenkabriebsammelvorrichtungsimplantat (38) derart angeordnet oder ausgebildet ist, um von der Synovialflüssigkeit (70) infolge einer Bewegung des Bewegungsgelenks durchströmt zu werden, insbesondere unidirektional oder bidirektional.

4. Medizinisches Gelenkabriebsammelvorrichtungsimplantat nach einem der voranstehenden Ansprüche, **dadurch gekennzeichnet, dass** das Gelenkabriebsammelvorrichtungsimplantat (38) eine Partikelextraktionseinrichtung (68) umfasst zum Extrahieren von Abriebpartikeln (52) aus der Synovialflüssigkeit (70),
wobei insbesondere die Partikelextraktionseinrichtung (68) im Sammelraum (50) angeordnet oder ausgebildet ist.

5. Medizinisches Gelenkabriebsammelvorrichtungsimplantat nach Anspruch 4, **dadurch gekennzeichnet, dass** die Partikelextraktionseinrichtung (68) eine Filtereinrichtung (72) und/oder eine strömungsmechanische Abscheideeinrichtung (74) umfasst.

6. Medizinisches Gelenkabriebsammelvorrichtungsimplantat nach Anspruch 5, **dadurch gekennzeichnet, dass** die Abscheideeinrichtung (74) in Form einer Trägheits- oder Umlenkabscheideeinrichtung ausgebildet ist.

7. Medizinisches Gelenkabriebsammelvorrichtungsimplantat nach Anspruch 5 oder 6, **dadurch gekennzeichnet, dass** die Abscheideeinrichtung (74) eine Mehrzahl von, insbesondere im Sammelraum (50) angeordneten oder ausgebildeten, Umlenkelementen (76) umfasst zum Ausbilden von Abscheidebereichen (78), in welchen eine Strömungsgeschwindigkeit der Synovialflüssigkeit (70) durch den Sammelraum (50) hindurch Null oder im Wesentlichen Null ist.

8. Medizinisches Gelenkabriebsammelvorrichtungsimplantat nach Anspruch 7, **dadurch gekennzeichnet, dass** mindestens ein Teil der Mehrzahl von Umlenkelementen (76)
a) in Form von Vorsprüngen (80) ausgebildet ist, welche insbesondere von der Sammelraumbegrenzungsfläche (58) vorstehend angeordnet oder ausgebildet sind,
und/oder
b) eine Partikelrückhaltestruktur aufweist oder mindestens ein Partikelrückhalteelement trägt oder umfasst.

9. Medizinisches Gelenkabriebsammelvorrichtungsimplantat nach einem der Ansprüche 5 bis 8, **dadurch gekennzeichnet, dass** die Filtereinrichtung (72) mindestens ein Filterelement (88) umfasst, welches ausgebildet ist, um Partikel (52), insbesondere Abriebpartikel, zurückzuhalten.

10. Medizinisches Gelenkabriebsammelvorrichtungsimplantat nach Anspruch 9, **dadurch gekennzeichnet, dass**
a) das mindestens eine Filterelement (88) eine Porengröße in einem Bereich von etwa 5 µm ECD bis etwa 100 µm ECD aufweist
und/oder
b) zwei oder mehr Filterelemente (88) vorgesehen sind, die sich in ihren Porengrößen unterscheiden,
und/oder
c) zwischen dem Sammelraumeinlass (54) und dem Sammelraumauslass (56) mehrere Filterelemente (88) nacheinander angeordnet oder ausgebildet sind und dass Porengrößen der nacheinander angeordneten Filterelemente (88) ausgehend vom Sammelraumeinlass (54) abnehmen.

11. Medizinisches Gelenkabriebsammelvorrichtungsimplantat nach einem der voranstehenden Ansprüche, **dadurch gekennzeichnet, dass** das Gelenkabriebsammelvorrichtungsimplantat (38) einen Wirkstoffbehälter (94) umfasst, welcher mit einem medizinischen und/oder pharmazeutischen Wirkstoff befüllt oder befüllbar ist,
wobei insbesondere der Wirkstoff entzündungshemmend ist.

12. Medizinisches Gelenkabriebsammelvorrichtungsimplantat nach einem der voranstehenden Ansprüche, **dadurch gekennzeichnet, dass** eine innere Oberfläche (96) des Sammelraums (50), insbesondere die Sammelraumbegrenzungsfläche (58), mit einer Beschichtung versehen ist zum chemischen und/oder elektrostatischen Binden von Abriebpartikeln (52).

13. Gelenkendoprothesenkomponente (12, 14), welche ausgebildet ist in Form einer ersten Gelenkendoprothesenkomponente (12) mit einer ersten Gelenkfläche (24) zur Ausbildung eines Bewegungsgelenks im Zusammenwirken mit einer zweiten Gelenkfläche (28) einer zweiten Gelenkendoprothesenkomponente (14) oder einer natürlichen Gelenkfläche eines natürlichen Gelenkknochens eines Menschen oder eines Tieres, **dadurch gekennzeichnet, dass** die erste Gelenkendoprothesenkomponente (12) mindestens ein, insbesondere nur ein, Gelenkabriebsammelvorrichtungsimplantat (38) nach einem der voranstehenden Ansprüche umfasst oder bildet.

14. Gelenkendoprothesenkomponente nach Anspruch 13, **dadurch gekennzeichnet, dass**
a) das Gelenkabriebsammelvorrichtungsimplantat (38) an der ersten Gelenkendoprothesenkomponente (12) räumlich getrennt von der ersten Gelenkfläche (24) angeordnet oder ausgebildet ist und/oder
b) an der ersten Gelenkfläche (24) weder ein Sammelraumeinlass (54) noch ein Sammelraumauslass (56) des Sammelraums (50) angeordnet oder ausgebildet sind,
und/oder
c) der Sammelraumeinlass (54) und der Sammelraumauslass (56) außerhalb der ersten Gelenkfläche (24) oder getrennt von dieser angeordnet oder ausgebildet sind
und/oder
d) das Gelenkabriebsammelvorrichtungsimplantat (38) an der ersten Gelenkendoprothesenkomponente (12) in einem Bereich geringer mechanischer Belastung angeordnet oder ausgebildet ist.

15. Gelenkendoprothese (10) umfassend eine erste Gelenkendoprothesenkomponente (12) und eine mit dieser zusammenwirkende zweite Gelenkendoprothesenkomponente (14) zur Ausbildung eines Bewegungsgelenks, **dadurch gekennzeichnet, dass** die erste und/oder die zweite Gelenkendoprothesenkomponente (12, 14) in Form einer Gelenkendoprothesenkomponente (12, 14) nach Anspruch 13 oder 14 ausgebildet sind, wobei insbesondere die Gelenkendoprothese (10) in Form eines Kniegelenks (16), eines Hüftgelenks, eines Schultergelenks, eines Sprunggelenks, eines Handgelenks, eines Bandscheibengelenks (102) oder eines Ellbogengelenks ausgebildet ist.

## Claims

1. Medical joint abrasion collection apparatus implant (38), which is configured to be implanted in a joint capsule (66) of a movement joint, which movement joint comprises at least one first artificial joint endoprosthesis component (12) with a first joint surface (24) for forming the movement joint in cooperation with a second joint surface (28) of a second artificial joint endoprosthesis component (14) or a natural joint surface of a natural joint bone of a human or an animal, wherein the joint abrasion collection apparatus implant (38) comprises an implant body (48) on which a collection space (50) is formed for receiving particles (52), in particular abrasion particles, that are released by abrasion on the at least one first joint endoprosthesis component (12) into the synovial fluid (70) contained in a joint capsule (66) of the movement joint, wherein the collection space (50) comprises a collection space inlet (54) and a collection space outlet (56) and, except for the collection space inlet (54) and the collection space outlet (56), is delimited by a fluid-tight, closed collection space delimiting surface (58), wherein the collection space inlet (54) is closed with a collection space inlet closure element (90) and/or the collection space outlet (56) is closed with a collection space outlet closure element (92), **characterized in that** the collection space inlet closure element (90) and the collection space outlet closure element (92) are made of a closure element material that is soluble in the synovial fluid (70).

2. Medical joint abrasion collection apparatus implant in accordance with any one of the preceding Claims, **characterized in that** the collection space (50) is of hollow-cylindrical or substantially hollow-cylindrical configuration, in particular in the form of a bore (60) passing through the joint abrasion collection apparatus implant (38).

3. Medical joint abrasion collection apparatus implant in accordance with any one of the preceding Claims, **characterized in that** the collection space (50) is arranged or formed on the joint abrasion collection apparatus implant (38) so as to be flowed through, in particular unidirectionally or bidirectionally, by the synovial fluid (70) as a result of a movement of the movement joint.

4. Medical joint abrasion collection apparatus implant in accordance with any one of the preceding Claims, **characterized in that** the joint abrasion collection apparatus implant (38) comprises a particle extraction device (68) for extracting abrasion particles (52) from the synovial fluid (70), wherein in particular the particle extraction device (68) is arranged or formed in the collection space (50).

5. Medical joint abrasion collection apparatus implant in accordance with Claim 4, **characterized in that** the particle extraction device (68) comprises a filter device (72) and/or a fluid mechanical separating device (74).

6. Medical joint abrasion collection apparatus implant in accordance with Claim 5, **characterized in that** the separating device (74) is configured in the form of an inertial or deflection separating device.

7. Medical joint abrasion collection apparatus implant in accordance with Claim 5 or 6, **characterized in that** the separating device (74) comprises a plurality of deflecting elements (76), in particular arranged or formed in the collection space (50), for forming separation regions (78) in which a flow speed of the synovial fluid (70) through the collection space (50) is zero or substantially zero.

8. Medical joint abrasion collection apparatus implant in accordance with Claim 7, **characterized in that** at least a portion of the plurality of deflecting elements (76)
a) is configured in the form of projections (80), which in particular are arranged or formed projecting from the collection space delimiting surface (58),
and/or
b) has a particle retaining structure or carries or comprises at least one particle retaining element.

9. Medical joint abrasion collection apparatus implant in accordance with any one of Claims 5 to 8, **characterized in that** the filter device (72) comprises at least one filter element (88), which is configured to retain particles (52), in particular abrasion particles.

10. Medical joint abrasion collection apparatus implant in accordance with Claim 9, **characterized in that**
a) the at least one filter element (88) has a pore size in a range of about 5 µm ECD to about 100 µm ECD
and/or
b) two or more filter elements (88) are provided, which differ in their pore sizes,
and/or
c) a plurality of filter elements (88) are arranged or formed one after the other between the collection space inlet (54) and the collection space outlet (56), and **in that** pore sizes of the filter elements (88) arranged one after the other decrease commencing from the collection space inlet (54).

11. Medical joint abrasion collection apparatus implant in accordance with any one of the preceding Claims, **characterized in that** the joint abrasion collection apparatus implant (38) comprises an active substance container (94), which is filled or fillable with a medical and/or pharmaceutical active substance,
wherein in particular the active substance is anti-inflammatory.

12. Medical joint abrasion collection apparatus implant in accordance with any one of the preceding Claims, **characterized in that** an inner surface (96) of the collection space (50), in particular the collection space delimiting surface (58), is provided with a coating for chemically and/or electrostatically binding abrasion particles (52).

13. Joint endoprosthesis component (12, 14), which is configured in the form of a first joint endoprosthesis component (12) with a first joint surface (24) for forming a movement joint in cooperation with a second joint surface (28) of a second joint endoprosthesis component (14) or a natural joint surface of a natural joint bone of a human or an animal, **characterized in that** the first joint endoprosthesis component (12) comprises or forms at least one, in particular only one, joint abrasion collection apparatus implant (38) in accordance with any one of the preceding Claims.

14. Joint endoprosthesis component in accordance with Claim 13, **characterized in that**
a) the joint abrasion collection apparatus implant (38) on the first joint endoprosthesis component (12) is arranged or formed spatially separated from the first joint surface (24)
and/or
b) neither a collection space inlet (54) nor a collection space outlet (56) of the collection space (50) is arranged or formed on the first joint surface (24),
and/or
c) the collection space inlet (54) and the collection space outlet (56) are arranged or formed outside the first joint surface (24) or separated therefrom
and/or
d) the joint abrasion collection apparatus implant (38) is arranged or formed on the first joint endoprosthesis component (12) in a region of low mechanical strain.

15. Joint endoprosthesis (10), comprising a first joint endoprosthesis component (12) and a second joint endoprosthesis component (14) cooperating therewith to form a movement joint, **characterized in that** the first and/or the second joint endoprosthesis component (12, 14) is/are configured in the form of a joint endoprosthesis component (12, 14) in accordance with Claim 13 or 14,
wherein in particular the joint endoprosthesis (10) is configured in the form of a knee joint (16), a hip joint, a shoulder joint, an ankle joint, a wrist joint, an intervertebral disc joint (102), or an elbow joint.

## Revendications

1. Implant médical de collecte de débris articulaires (38), qui est conçu pour être implanté dans une capsule articulaire (66) d'une articulation mobile, laquelle articulation mobile comprend au moins une première composante d'endoprothèse articulaire artificielle (12) avec une première surface articulaire (24) destinée à former l'articulation mobile en coopération avec une deuxième surface articulaire (28) d'une deuxième composante d'endoprothèse articulaire artificielle (14) ou une surface articulaire naturelle d'un os articulaire naturel d'un être humain ou d'un animal, l'implant médical de collecte de débris articulaires (38) comprenant un corps d'implant (48), sur lequel est formée une chambre de collecte (50) destinée à recevoir des particules (52), en particulier des particules d'usure, qui sont libérées par l'usure sur au moins la première composante d'endoprothèse articulaire (12) dans le liquide synovial (70) contenu dans une capsule articulaire (66) de l'articulation mobile, la chambre de collecte (50) comprenant une entrée de chambre de collecte (54) et une sortie de chambre de collecte (56) et étant, à l'exception de l'entrée de chambre de collecte (54) et de la sortie de chambre de collecte (56), délimitée par une surface de délimitation de chambre de collecte (58) étanche aux fluides et fermée, l'entrée de chambre de collecte (54) étant fermée par un élément de fermeture d'entrée de chambre de collecte (90) et/ou la sortie de chambre de collecte (56) étant fermée par un élément de fermeture de sortie de chambre de collecte (92), **caractérisé en ce que** l'élément de fermeture d'entrée de chambre de collecte (90) et l'élément de fermeture de sortie de chambre de collecte (92) sont constitués d'un matériau d'élément de fermeture qui est soluble dans le liquide synovial (70).

2. Implant médical de collecte de débris articulaires selon l'une des revendications précédentes, **caractérisé en ce que** la chambre de collecte (50) est formée de manière cylindrique creuse ou essentiellement cylindrique creuse, en particulier sous la forme d'un perçage (60) traversant l'implant médical de collecte de débris articulaires (38).

3. Implant médical de collecte de débris articulaires selon l'une des revendications précédentes, **caractérisé en ce que** la chambre de collecte (50) est disposée ou formée sur l'implant médical de collecte de débris articulaires (38) de telle sorte qu'elle soit traversée par le liquide synovial (70) à la suite d'un mouvement de l'articulation mobile, en particulier de manière unidirectionnelle ou bidirectionnelle.

4. Implant médical de collecte de débris articulaires selon l'une des revendications précédentes, **caractérisé en ce que** l'implant médical de collecte de débris articulaires (38) comprend un dispositif d'extraction de particules (68) pour extraire des particules d'usure (52) du liquide synovial (70),
le dispositif d'extraction de particules (68) étant en particulier disposé ou formé dans la chambre de collecte (50).

5. Implant médical de collecte de débris articulaires selon la revendication 4, **caractérisé en ce que** le dispositif d'extraction de particules (68) comprend un dispositif de filtration (72) et/ou un dispositif de séparation hydrodynamique (74).

6. Implant médical de collecte de débris articulaires selon la revendication 5, **caractérisé en ce que** le dispositif de séparation (74) est formé sous la forme d'un dispositif de séparation par inertie ou de déviation.

7. Implant médical de collecte de débris articulaires selon la revendication 5 ou 6, **caractérisé en ce que** le dispositif de séparation (74) comprend une pluralité d'éléments de déviation (76), en particulier disposés ou formés dans la chambre de collecte (50), pour former des zones de séparation (78) dans lesquelles une vitesse d'écoulement du liquide synovial (70) à travers la chambre de collecte (50) est nulle ou essentiellement nulle.

8. Implant médical de collecte de débris articulaires selon la revendication 7, **caractérisé en ce qu'**au moins une partie de la pluralité d'éléments de déviation (76)
a) est formée sous la forme de saillies (80), qui sont en particulier disposées ou formées en saillie à partir de la surface de délimitation de la chambre de collecte (58),
et/ou
b) présente une structure de rétention de particules ou porte ou comprend au moins un élément de rétention de particules.

9. Implant médical de collecte de débris articulaires selon l'une des revendications 5 à 8, **caractérisé en ce que** le dispositif de filtration (72) comprend au moins un élément filtrant (88) qui est conçu pour retenir des particules (52), en particulier des particules d'usure.

10. Implant médical de collecte de débris articulaires selon la revendication 9, **caractérisé en ce que**
a) le ou les éléments filtrants (88) présentent une taille de pores dans une plage d'environ 5 µm ECD jusqu'à environ 100 µm ECD
et/ou
b) deux ou plusieurs éléments filtrants (88) sont prévus, qui diffèrent par leur taille de pores,
et/ou
c) plusieurs éléments filtrants (88) sont disposés ou formés successivement entre l'entrée de la chambre de collecte (54) et la sortie de la chambre de collecte (56), et que les tailles de pores des éléments filtrants (88) disposés successivement diminuent à partir de l'entrée de la chambre de collecte (54).

11. Implant médical de collecte de débris articulaires selon l'une des revendications précédentes, **caractérisé en ce que** l'implant médical de collecte de débris articulaires (38) comprend un réservoir d'agent actif (94), qui est rempli ou remplissable d'un agent actif médical et/ou pharmaceutique,
l'agent actif étant en particulier anti-inflammatoire.

12. Implant médical de collecte de débris articulaires selon l'une des revendications précédentes, **caractérisé en ce qu'**une surface interne (96) de la chambre de collecte (50), en particulier la surface de délimitation de la chambre de collecte (58), est pourvue d'un revêtement destiné à la liaison chimique et/ou électrostatique de particules d'usure (52).

13. Composante d'endoprothèse articulaire (12, 14), qui est conçue sous la forme d'une première composante d'endoprothèse articulaire (12) avec une première surface articulaire (24) pour former une articulation mobile en coopération avec une deuxième surface articulaire (28) d'une deuxième composante d'endoprothèse articulaire (14) ou avec une surface articulaire naturelle d'un os articulaire naturel d'un être humain ou d'un animal, **caractérisé en ce que** la première composante d'endoprothèse articulaire (12) comprend ou forme au moins un, et en particulier un seul, implant médical de collecte de débris articulaires (38), selon l'une des revendications précédentes.

14. Composant de prothèse articulaire selon la revendication 13, **caractérisé en ce que**
a) l'implant médical de collecte de débris articulaire (38) est disposé ou formé sur la première composante d'endoprothèse articulaire (12) spatialement séparé de la première surface articulaire (24)
et/ou
b) ni une entrée de chambre de collecte (54) ni une sortie de chambre de collecte (56) de la chambre de collecte (50) ne sont disposées ou formées sur la première surface articulaire (24),
et/ou
c) l'entrée de chambre de collecte (54) et la sortie de chambre de collecte (56) sont disposées ou formées à l'extérieur de la première surface articulaire (24) ou séparément de celle-ci
et/ou
d) l'implant médical de collecte de débris articulaire (38) est disposé ou formé sur la première composante d'endoprothèse articulaire (12) dans une zone de faible sollicitation mécanique.

15. Endoprothèse articulaire (10) comprenant une première composante d'endoprothèse articulaire (12) et une deuxième composante d'endoprothèse articulaire (14) coopérant avec celui-ci pour former une articulation mobile, **caractérisée en ce que** la première et/ou la deuxième composante d'endoprothèse articulaire (12, 14) sont réalisées sous la forme d'une composante d'endoprothèse articulaire (12, 14) selon la revendication 13 ou 14,
l'endoprothèse articulaire (10) étant en particulier réalisée sous la forme d'une articulation du genou (16), d'une articulation de la hanche, d'une articulation de l'épaule, d'une articulation de la cheville, d'une articulation du poignet, d'une articulation de disque intervertébral (102) ou d'une articulation du coude.
